# EUROPEAN PATENT APPLICATION

(11) **EP 4 620 962 A1**
(43) Date of publication of application: **24.09.2025**
(21) Application number: 23891876.7
(22) Date of filing: 01.11.2023
(51) Int. Cl.: C07K 5/11, C07K 5/072, A61K 8/64, A61Q 7/00, A61P 17/14, A61K 38/00

(54) **PEPTIDE HAVING ACTIVITIES TO PROMOTE HAIR GROWTH AND AMELIORATE DAMAGED HAIR, AND USE THEREOF**

(30) Priority: 18.11.2022 KR 20220155815
(71) Applicant: Caregen Co., Ltd., Anyang-si, Gyeonggi-do 14119 (KR)
(72) Inventor: CHUNG, Yong Ji, Anyang-si, Gyeonggi-do 14119 (KR); KIM, Eun Mi, Anyang-si, Gyeonggi-do 14119 (KR)
(74) Representative: Potter Clarkson
(86) International application number: PCT/KR2023/017241
(87) International publication number: WO 2024/106809

(57) **Abstract**

Provided are a peptide having hair growth promoting and damaged hair improving activity and uses thereof. Provided are a peptide consisting of an amino acid sequence represented by Arg-Cys-Cys-Gly or Glu-Glu, a cosmetic composition for improving condition of damaged hair including the peptide, a cosmetic composition for alleviating hair loss or promoting hair growth including the peptide, and a pharmaceutical composition for preventing or treating hair loss including the peptide.

## Description

### Technical Field

The disclosure relates to a peptide having hair growth promoting and damaged hair improving activity, and uses thereof.

### Background Art

A human scalp has about 150,000 hairs, each going through a different hair cycle, and a process of growing and falling out is repeated. A hair growth cycle is broadly divided into 3 phases: anagen as a growth phase in which the hair grows, catagen as a regressing phase in which the hair stops growing and remains, and telogen as a resting phase in which dermal papilla cells contract and a hair follicle shrinks causing hair loss. These three phases are periodically repeated. The hair growth cycle varies according to internal factors such as family history, genetic factors, physical constitution, and hormonal regulation, and external factors such as nutritional status, aging, surrounding environment, and stress.

Meanwhile, because end portions of hairs are exposed to an external environments for a longer period of time than portions close to the scalp, more severe damage is caused in the end portions by the external environment. Weathering caused by exposure to the external environment results in loss of cuticles of hair, split ends, and eventually breakage. Continuous application of heat to hair by using hair dryer, or the like is a representative example of the environments causing hair damage such as a decrease in hair tension and weakened hair. Also, various physical, chemical, or environmental factors such as frequent hair perms and hair dyes may cause elution of proteins and lipids from the hair causing hair porousness. As a result, hair becomes rough and losses gloss, and increased friction makes hair care difficult, resulting in a phenomenon of broken hair and split ends. In addition, by such hair porousness, elasticity of hair is decreased and hair sags, causing problems of reduction in hair volume and hair thinning.

As an aging society develops, the number of people suffering from hair loss is increased. Hair loss was previously a major concern for middle-aged men, but in recent years, an interest in hair loss prevention and hair growth is increasing among young people and women as well. Hair loss has been recognized as a series of aging phenomena, but it has been recently found that hair loss progresses due to various causes including stress, westernized eating habits, nutritional imbalance, and changes in social activities, along with genetic factors. As drugs that exhibit hair-generating effects and are approved by the US FDA, Minoxidil (6-Amino-1,2-dihydro-1-hydroxy-2-imino-4-phenoxypyrimidine) (US Patent No. 3,382,247) and Finasteride (US Patent No. 5,215,894) are currently available . In the case of Minoxidil, it was developed as a vasodilator for the treatment of hypertension in the early 1970s. However, as a side effect of hirsutism was reported, it has been used as a hair growth promotor. Accordingly, phenomena of exhibiting effects of thickening the hair by thickened hair follicles and increased diameter of the hair are shown. Also, in the case of finasteride, it was developed as a medicine for benign prostatic hyperplasia, but is currently used as a medicine for hair loss. Accordingly, phenomena of slowing down the hair loss progress and exhibiting hair-generating effects are shown. However, Minoxidil had side effects reported such as weight gain, edema, dermatitis, and heart rate increase, and finasteride which requires continuous use had side effects such as sexual dysfunction in men and birth defects in pregnant women. Thus, the development of hair loss treatment without side effects is still in demand.

Under such technical background, various studies are in progress to improve condition of damaged hair and prevent hair loss through mechanisms including hormone control and metabolic control (Korean Patent Application Publication No. 2002-0005332), but results thereof are still insignificant.

### Disclosure of Invention

### Technical Problem

Provided is a peptide consisting of an amino acid sequence represented by Arg (R)-Cys (C)-Cys (C)-Gly (G) or Glu (E)-Glu (E).

Provided is a cosmetic composition for improving condition of damaged hair including a peptide including an amino acid sequence represented by Arg (R)-Cys (C)-Cys (C)-Gly (G) or Glu (E)-Glu (E) as an active ingredient.

Provided is a cosmetic composition for alleviating hair loss or promoting hair growth including a peptide including an amino acid sequence represented by Arg (R)-Cys (C)-Cys (C)-Gly (G) or Glu (E)-Glu (E) as an active ingredient.

Provided is a pharmaceutical composition for preventing or treating hair loss including a peptide including an amino acid sequence represented by Arg (R)-Cys (C)-Cys (C)-Gly (G) or Glu (E)-Glu (E) as an active ingredient.

Other objects and advantages of the present application will become more apparent by the following detailed description in conjunction with the appended claims and drawings. Contents not described in the present specification will be omitted because they can be sufficiently recognized and inferred by a person skilled in the art of the present application or in a similar technical field.

### Solution to Problem

Description and embodiments disclosed herein may also be applied to other descriptions and embodiments, respectively. That is, all combinations of various elements disclosed herein belong to the scope of the present disclosure. In addition, the scope of the present application is not construed to be limited by the detailed description provided below.

According to an aspect of the disclosure, a peptide consists of an amino acid sequence represented by Arg (R)-Cys (C)-Cys (C)-Gly (G) or Glu (E)-Glu (E).

As used herein, the term "peptide" refers to a linear molecule formed of amino acid residues linked by peptide bonds. The peptide may be prepared by any known chemical synthesis methods, particularly, solid-phase synthesis techniques (Merrifield, J. Amer. Chem. Soc. 85:2149-54(1963); Stewart, et al., Solid Phase Peptide Synthesis, 2nd. ed., Pierce Chem. Co.: Rockford, 111(1984)) or liquid-phase synthesis techniques (US Patent No. 5,516,891). As a result of intensive efforts to develop peptides having biologically effective activity, the present inventors have found a peptide having an amino acid sequence represented by Arg (R)-Cys (C)-Cys (C)-Gly (G) or Glu (E)-Glu (E). In this regard, the biologically effective activity may exhibit at least one characteristic selected from (a) promoting proliferation or activation of hair follicle dermal papilla cells; (b) promoting activation of outer root sheath cells; (c) promoting activation of germinal matrix cells; and (d) inhibiting expression of Dickkopf-related protein 1 (DKK-1). More specifically, the biologically effective activity may be (aa) enhancement of phosphorylation of ERF and AKT, which are factors related to proliferation of hair follicle dermal papilla cells; (bb) activation of beta-catenin, lymphoid enhancer-binding factor-1 (LEF-1), c-Myc, and cyclin D1 of hair follicle dermal papilla cells; (cc) enhancement of expression of type I cuticular Ha3-II (Ha3-II), Keratin 5, Keratin 14, and Keratin 19 of outer root sheath cells; and (dd) enhancement of expression of Homeobox protein Hox-C13 (HOXC13), Msh homeobox 2 (MSX2), and Forkhead box protein N1 (FOXN1) of germinal matrix cells. Therefore, the peptide may be used to alleviate hair loss or promote hair growth, to prevent or treat hair loss, and to improve condition of damaged hair.

A protective group may be bound to a N- or C-terminal of the peptide to obtain chemical stability, enhanced pharmacological properties (such as half-life, absorption, potency, and efficacy), modified specificity (such as broad spectrum of biological activity), and reduced antigenicity. Therefore, the peptide may be used as both the peptide itself or a protected derivative thereof. According to an embodiment, the N-terminal of the peptide may be bound to one protective group selected from an acetyl group, a fluoreonylmethoxycarbonyl group, a formyl group, a palmitoyl group, a myristyl group, a stearyl group, a butoxycarbonyl group (Boc), an allyloxycarbonyl group (Alloc), and a polyethylene glycol (PEG); and/or the C-terminal of the peptide may be bound to one protective group selected from an amino group (-NH₂), a tertiary alkyl group, and an azide group (-NHNH₂). In addition, the peptide may optionally include a targeting sequence, a tag, a labeled residue, and an amino acid sequence constructed for a specific purpose of increasing half-life or peptide stability.

The peptide may be an artificially synthesized or non-naturally occurring or engineered, and the "non-naturally occurring or engineered" refers to a state created by applying artificial modifications, rather than a state as it exists in nature. In this regard, the artificial modification may include artificial synthesis of an amino acid sequence by mimicking a plurality of amino acid structures, or engineering to obtain chemical stability, enhanced pharmacological properties, modified specificity, or reduced antigenicity as described above.

As used herein, the term "stability" may refer to not only *in vivo* stability of protecting the peptide from an attack of a protease in a living body, but also storage stability (storage stability at room temperature).

According to another aspect of the disclosure, a cosmetic composition for improving condition of damaged hair includes a peptide including an amino acid sequence represented by Arg (R)-Cys (C)-Cys (C)-Gly (G) or Glu (E)-Glu (E) as an active ingredient.

As used herein, the term "improving condition" may refer to all actions that increases parameters related to amelioration or treatment of symptoms, for example, at least decrease degrees of symptoms.

As used herein, the term "damaged hair" may refer to hair modified or weakened by a physical, chemical, or environmental factor. The damaged hair may be, for example, hair in which a gap between cuticles constituting hair is widened or hair from which cuticles are partially fallen off. In addition, the damaged hair may refer to hair with rough overall texture and reduced elasticity and volume as roughness and friction increase while tension and gloss decrease compared to normal hair. In addition, the damaged hair may refer to hair in which bonds between cysteines are broken by a physical or chemical stimulus from the outside.

As used herein, the term "improving condition of damaged hair" may refer restoring the state of the damaged hair described above to a state of normal hair. The improving condition of damaged hair may refer to, for example, improving elasticity (tension) and gloss of the hair, reducing roughness and friction of hair, and restoring cuticles in hair. Also, the improving condition of damaged hair may mean reconnecting or restoring inter-cysteine bonds that have been broken by external stimuli. Also, the term may be used interchangeably with "promoting regeneration of damaged hair" and "improving hair protection function".

Although conventional functional peptides have effective biological activity, the peptides were not effectively introduced into target tissues or cells due to large sizes thereof or the peptide disappears within a short period of time due to short half-lives thereof. However, because the cosmetic composition according to an embodiment includes a peptide consisting of 10 or less amino acids as an active ingredient, penetrability of the active ingredient into skin or hair is very high, and for example, a damaged state of hair may be restored thereby in the case of being topically applied to a certain area.

According to an embodiment, the peptide exhibited efficacy such as promoting proliferation or activation of hair follicle dermal papilla cells, promoting activation of outer root sheath cells, and promoting activation of germinal matrix cells. In addition, in a test using human hair or human hair wigs, the peptide exhibited effects on enhancement of tension and gloss of damaged hair and reduction in roughness and friction of damaged hair, and excellent skin safety was confirmed. That is, the above-described efficacy verifies effects on promoting enhancement of condition of damaged hair/regeneration of damaged hair and improving hair protection function. The peptide may be used as an active ingredient of a cosmetic composition for improving condition of damaged hair.

According to an embodiment, the active ingredient of the cosmetic composition, e.g., the functional cosmetic composition, may include the peptide itself having the above-described biological activity, a protected derive thereof, of a peptide including the amino acid sequence of the peptide as a basic backbone and extended end portions (e.g., 10-mer, 9-mer, 8-mer, 7-mer, 6-mer, and 5-mer peptides).

The cosmetic composition may include a cosmetologically effective amount of the peptide; and/or a cosmetically acceptable carrier, but is not limited thereto.

As used herein, the term "cosmetologically effective amount" may refer to an amount sufficient to achieve the effects of the cosmetic composition on alleviating hair loss or promoting hair growth.

A weight ratio of the peptide to the cosmetically acceptable carrier may be, for example, from about 500:1 to about 1:500, for example, the weight ratio may be from about 450:1 to about 1:450, from about 400:1 to about 1:400, from about 350:1 to about 1:350, from about 300:1 to about 1:300, from about 250:1 to about 1:250, from about 200:1 to about 1:200, from about 150:1 to about 1:150, from about 100:1 to about 1:100, from about 80:1 to about 1:80, from about 60:1 to about 1:60, from about 40:1 to about 1:40, from about 20:1 to about 1:20, from about 10:1 to about 1:10, from about 8:1 to about 1:8, from about 6:1 to about 1:6, from about 4:1 to about 1:4, or from about 2:1 to about 1:2, but is not limited thereto.

The cosmetic composition may be prepared as any formulation commonly produced in the art, for example, solutions, suspensions, emulsions, pastes, gels, creams, lotions, mists, powders, soaps, oils, powder foundations, emulsion foundations, wax foundations and sprays, but is not limited thereto. The cosmetic composition may be prepared as a formulation selected from scalp clinic agents, scalp scaling agents, scalp massaging agents, scalp care products, cleansers, shampoos, hair tonics, hair conditioners, hair lotions, hair gels, hair packs, hair masks, hair essences, ointments, hair styling agents, hair dyes, and hair perm agents, but is not limited thereto.

When the formulation of the cosmetic composition is paste, cream, or gel, animal oil, vegetable oil, wax, paraffin, starch, tragacanth, cellulose derivative, polyethylene glycol, silicone, bentonite, talc, or zinc oxide may be used as a carrier.

When the formulation of the cosmetic composition is powder or spray, lactose, talc, silica, aluminum hydroxide, calcium silicate, or polyamide powder may be used as a carrier. For example, in the case of spray, the cosmetic composition may further include a propellent such as chlorofluorohydrocabon, propane/butane, or dimethyl ether.

When the formulation of the cosmetic composition is a solution or an emulsion, a solvent, a solubilizing agent, or an emulsifying agent may be used as a carrier and the cosmetic composition may include, for example, water, ethanol, isopropanol, ethyl carbonate, ethyl acetate, benzyl alcohol, benzyl benzoate, propylene glycol, 1,3-butylglycol oil, glycerol aliphatic ester, polyethylene glycol, or sorbitan fatty acid ester.

When the formulation of the cosmetic composition is a suspension, a liquid diluent such as water, ethanol, or propylene glycol, a suspending agent such as ethoxylated isostearyl alcohol, polyoxyethylene sorbitol ester, and polyoxyethylene sorbitan ester, microcrystalline cellulose, aluminum metahydroxide, bentonite, agar, or tragacanth may be used as a carrier.

The peptide may be contained in nanosomes or nanoparticles to further improve skin penetration or stability. For example, the nanosomes may be prepared by using lecithin as a raw ingredient with a microfluidizer, and contained in lecithin particles. Any known methods used to prepare nanosomes may be used therefor. The nanosomes may have a particle size of about 30 nm to about 200 nm. When the particle size of the nanosomes is less than 30 nm, skin penetration progresses very quickly to cause side effects on the skin. When the particle size of the nanosomes is greater than 200 nm, skin penetration does not easily occur making it difficult to obtain the effects of the nanosomes.

In addition to the peptide, as an active ingredient, and the carrier contained in the cosmetic composition, the cosmetic composition may further include, any adjuvants commonly used in cosmetic compositions, such as an antioxidant, a stabilizer, a solubilizer, a vitamin, a pigment, and a flavoring agent.

As an active ingredient contained in the cosmetic composition, the peptide may be included in an amount selected appropriately and non-limitedly according to a form or a product and a use desired to obtain, for example, in an amount of about 0.01 wt% to about 15 wt% based on a total weight of the entire cosmetic composition. In addition, for example, the cosmetic composition may include the peptide in an amount of 0.2 wt%, 0.5 wt%, about 1.0 wt% to about 3.0 wt%, preferably, about 2.0 wt% to about 3.0 wt% based on a total weight. The cosmetic composition may include, for example, about 0.1 to about 1.5 wt%, e.g., about 0.2 to about 1.0 wt%, about 0.2 to about 0.8 wt%, or about 0.2 to about 0.5 wt% of the peptide, about 0.2 to about 0.5 wt% of a surfactant, 0.01 to about 0.1 wt% of a pH regulator, and/or about 0.2 to about 0.5 wt% of a preservative, and optionally, about 4.0 to about 6.0 wt% of an emulsifier based on a total weigh to the entire cosmetic composition.

According to another aspect of the disclosure, a cosmetic composition for alleviating hair loss or promoting hair growth includes a peptide including an amino acid sequence represented by Arg (R)-Cys (C)-Cys (C)-Gly (G) or Glu (E)-Glu (E) as an active ingredient.

Terms or elements identical to those given above in the descriptions of the peptide are as described above.

As used herein, the term "alleviating hair loss" collectively refers to a process of treating, decreasing, and ameliorating a state of hair loss or effects thereof, and, for example, may refer to any action to inhibit the progress of hair loss, such as, to promote proliferation or activation of hair follicle dermal papilla cells, to promote activation of outer root sheath cells, to promote activation of germinal matrix cells, and to inhibit expression of a hair loss inducer such as DKK-1. The hair follicle dermal papilla cells, the outer root sheath cells, and the germinal matrix cells are cells constituting hair follicles affecting thickness and growth of hair. Activation of these cells affects the hair growth cycle and hair loss alleviating effects may be induced by this mechanism.

As used herein, the term "promoting hair growth" may refer to all actions to increase hair growth in hair follicles, for example, and for example, all actions to increase a total volume of hair such as promoting effects on proliferation of hair follicle cells, activation of hair follicle cells, or growth of hair follicle cells. Specifically, the hair growth cycle may be divided into anagen as a growth phase in which the hair grows, catagen as a regressing phase in which the hair stops growing and hair burb shrinks, telogen as a resting phase in which dermal papilla cells stop acting and hair stays on the scalp, and an active period in which dermal papilla cells start acting or old hair are shed by growing new hair. In the hair growth cycle, the dermal papilla cells are connected to capillaries and sensory nerves serving to supply oxygen and nutrients to the germinal matrix cells, the germinal matrix cells surround the dermal papilla cells serving to determine the growth rate by constant cell division and proliferation. In addition, the outer root sheath cells are cells located in an area in contact with a basal layer of epidermis and serve to protect hair until hair keratinization is completed.

According to an embodiment, the peptide exhibited effects on promoting proliferation or activation of hair follicle dermal papilla cells, promoting activation of outer root sheath cells, promoting activation of germinal matrix cells, and inhibiting expression of a hair loss inducer such as DKK-1. Therefore, the peptide may be used as an active ingredient of the cosmetic composition for alleviating hair loss or promoting hair growth.

According to another aspect of the disclosure, a method of improving condition of damaged hair, a method of alleviating hair loss, or a method of promoting hair growth, each including smearing a cosmetic composition including a peptide including an amino acid sequence represented by Arg (R)-Cys (C)-Cys (C)-Gly (G) or Glu (E)-Glu (E) as an active ingredient on skin or hair of an individual.

Terms or elements identical to those given above in the descriptions of the cosmetic composition are as described above.

As used herein, the terms "applying", "administering", and "smearing" are used interchangeably and may refer to causing at least partial localization of the composition of an embodiment to a desired position or disposing the composition of an embodiment into an individual via a route of administration.

According to another aspect of the disclosure, a pharmaceutical composition for preventing or treating hair loss includes a peptide including an amino acid sequence represented by Arg (R)-Cys (C)-Cys (C)-Gly (G) or Glu (E)-Glu (E) as an active ingredient.

Terms or elements identical to those given above in the descriptions of the peptide are as described above.

As used herein, the term "prevention" refers to all actions intended to inhibit or delay development of a disease by administering the composition.

As used herein, the term "treatment" refers to any form of treatment for an individual suffering from a disease or at risk of developing a disease to provide effects on improve the status (e.g., at least one symptom) of the individual, delaying the progression of the disease, delaying occurrence of symptoms, or slowing down the progression of symptoms. Therefore, the "treatment" and "prevention" are not intended to imply cure or complete elimination of symptoms.

The "individual" refers to a subject in need of treatment of a disease, and more specifically, mammals such as human or no-human primates, mice, dogs, cats, horses, and cattle.

The "hair loss", as a disease to be prevented or treated by the pharmaceutical composition, refers to a state in which hairs are absent in areas where hairs should normally exist and may means that grown hairs (thick and black hairs) fall out from the scalp. Although causes of hair loss are not limited, hair loss may be caused by genetic factors, hormonal imbalance, mental stress, exposure to air pollution, various eating habits such as intake of processed food, and environmental influences. For example, hair loss may be hereditary androgenetic alopecia (bald head), alopecia areata, tinea capitis caused by fungal infection, telogen alopecia, trichotillomania, hair growth disorders, and the like. Cicatricial alopecia that is scarring hair loss may refer to hair loss caused by lupus, folliculitis decalvans, lichen planopilaris, and hair loss caused by burn and external injury.

According to an embodiment, the peptide had effects on promoting proliferation or activation of hair follicle dermal papilla cells, promoting activation of outer root sheath cells, promoting activation of germinal matrix cells, and inhibiting expression of a hair loss inducer such as DKK-1. Therefore, the peptide may be used as an active ingredient of a pharmaceutical composition for preventing or treating hair loss.

The pharmaceutical composition may include the above-described peptide having biological activity, a protected derivative thereof, or a peptide including the amino acid sequence of the peptide as a basic backbone and extended end portions (e.g., 10-mer, 9-mer, 8-mer, 7-mer, 6-mer, and 5-mer peptides) as an active ingredient.

The pharmaceutical composition may include a pharmaceutically effective amount of the peptide; and/or a pharmaceutically acceptable carrier, without being limited thereto.

As used herein, the term "pharmaceutically effective amount" refers to an amount sufficient to achieve preventive or therapeutic efficacy of the pharmaceutical composition on hair loss.

The pharmaceutically acceptable carrier is commonly used to prepare formulations and may include lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, or mineral oil, but is not limited thereto. Pharmaceutically acceptable carriers and agents suitable in the present disclosure are described in detail in Remington's Pharmaceutical Sciences (19th ed., 1995).

A weight ratio of the peptide to the pharmaceutically acceptable carrier may be, for example, from about 500:1 to about 1:500, for example, the weight ratio may be from about 450:1 to about 1:450, from about 400:1 to about 1:400, from about 350:1 to about 1:350, from about 300:1 to about 1:300, from about 250:1 to about 1:250, from about 200:1 to about 1:200, from about 150:1 to about 1:150, from about 100:1 to about 1:100, from about 80:1 to about 1:80, from about 60:1 to about 1:60, from about 40:1 to about 1:40, from about 20:1 to about 1:20, from about 10:1 to about 1:10, from about 8:1 to about 1:8, from about 6:1 to about 1:6, from about 4:1 to about 1:4, or from about 2:1 to about 1:2, but is not limited thereto.

The pharmaceutical composition may further include a lubricant, a humectant, a sweetener, a flavoring agent, an emulsifier, a suspension, or a preservative in addition to the above-described ingredients, without being limited thereto.

The pharmaceutical composition may be administered orally or parenterally, preferably, parenterally. In case of parenteral administration, intramuscular injection, intravenous injection, subcutaneous injection, intraperitoneal injection, topical administration, transdermal administration, and the like may be used, without being limited thereto.

A daily dosage of the pharmaceutical composition may be from about 0.0001 µg to about 1000 µg, from about 0.001 µg to about 1000 µg, from about 0.01 µg to about 1000 µg, from about 0.1 µg to about 1000 µg, or from about 1.0 µg to about 1000 µg, but is not limited thereto, and may be prescribed in various manners based on factors such as formulation method, administration method, age, body weight, gender, and pathological condition of a patient, food, administration time, administration route, excretion rate, and sensitivity.

The pharmaceutical composition may be formulated by using a pharmaceutically acceptable carrier and/or excipient into a single-dose dosage form or a unit dosage form in a multidose container according to a method that may be easily carried out by those skilled in the art to which the present disclosure pertains.

The formulation may be a solution in oil or aqueous medium, a suspension or emulsion, an extract, a powder, a granule, a tablet, or a capsule and may further include a dispersant and/or a stabilizer.

According to another aspect of the disclosure, a method of preventing or treating hair loss includes administering a pharmaceutical composition including a therapeutically effective amount of a peptide including an amino acid sequence represented by Arg (R)-Cys (C)-Cys (C)-Gly (G) or Glu (E)-Glu (E) as an active ingredient to an individual.

Terms or elements identical to those given above in the descriptions of the pharmaceutical composition are as described above.

According to another aspect of the disclosure, a food composition for improving condition of damaged hair, preventing hair loss, or promoting hair growth includes a peptide including an amino acid sequence represented by Arg (R)-Cys (C)-Cys (C)-Gly (G) or Glu (E)-Glu (E) as an active ingredient.

Terms or elements identical to those given above in the descriptions of the peptide are as described above.

The content of the peptide contained in the food composition as an active ingredient may be appropriately selected without limitation according to type of food, intended use, and the like, and may be, for example, from about 0.01 wt% to about 15 wt% based on a total weight of the food. In addition, for example, the content of the peptide may be from about 0.02 g to about 10 g, preferably, from about 0.3 g to about 1 g based on 100 ml of a health drink composition.

### Advantageous Effects of Invention

The peptide according to an embodiment may be applied to improve condition of damaged hair, alleviate hair loss, generate hair, and promote hair growth, because the peptide promotes proliferation and activation of dermal papilla cells, germinal matrix cells, and outer root sheath cells, which constitute hair follicles, and inhibits expression of DKK-1, which is a hair loss-related factor.

The peptide according to an embodiment may be used as an active ingredient of compositions for improving condition of damaged hair, alleviating hair loss, or promote hair growth.

### Brief Description of Drawings

FIG. 1 shows results of identifying cells proliferation levels after adding Peptide-1 according to an embodiment to hair follicle dermal papilla cells.
FIG. 2 shows results of identifying cells proliferation levels after adding Peptide-2 according to an embodiment to hair follicle dermal papilla cells.
FIG. 3 shows results of identifying phosphorylation of ERF and AKT, which are factors related to proliferation of hair follicle dermal papilla cells, after adding Peptide-1 according to an embodiment to hair follicle dermal papilla cells.
FIG. 4 shows results of identifying phosphorylation of ERF and AKT, which are factors related to proliferation of hair follicle dermal papilla cells, after adding Peptide-2 according to an embodiment to hair follicle dermal papilla cells.
FIG. 5 shows results of identifying migration of beta-catenin, which is a factor related to proliferation of hair follicle dermal papilla cells, from cytoplasm to a nucleus after adding Peptide-1 according to an embodiment to hair follicle dermal papilla cells.
FIG. 6 shows results of identifying migration of beta-catenin, which is a factor related to proliferation of hair follicle dermal papilla cells, from cytoplasm to a nucleus after adding Peptide-2 according to an embodiment to hair follicle dermal papilla cells.
FIG. 7 shows results of identifying expression levels of c-Myc and cyclin D1, which are downstream target genes of beta-catenin activation mechanism, after adding Peptide-1 according to an embodiment to hair follicle dermal papilla cells.
FIG. 8 shows results of identifying expression levels of LEF-1, c-Myc, and cyclin D1, which are downstream target genes of beta-catenin activation mechanism, after adding Peptide-2 according to an embodiment to hair follicle dermal papilla cells.
FIG. 9 shows results of identifying expression levels of DKK-1, which is a hair loss inducer, after adding Peptide-1 according to an embodiment to hair follicle dermal papilla cells.
FIG. 10 shows results of identifying expression levels of DKK-1, which is a hair loss inducer, after adding Peptide-2 according to an embodiment to hair follicle dermal papilla cells.
FIG. 11 shows results of identifying expression levels of Ha3-II, Keratin 14, and Keratin 19, which are activators of outer root sheath cells, after adding Peptide-1 according to an embodiment to outer root sheath cells.
FIG. 12 shows results of identifying expression levels of Ha3-II, Keratin 5, Keratin 14, and Keratin 19, which are activators of outer root sheath cells, after adding Peptide-2 according to an embodiment to outer root sheath cells.
FIG. 13 shows results of identifying expression levels of HOXC13, MSX2, and FOXN1, which are activators of germinal matrix cells, after adding Peptide-1 according to an embodiment to germinal matrix cells.
FIG. 14 shows results of identifying expression levels of MSX2 and FOXN1, which are activators of germinal matrix cells, after adding Peptide-2 according to an embodiment to germinal matrix cells.
FIG. 15 shows results of measuring tensions of hair over time, after applying a composition according to an embodiment (Example 1) to human hair.
FIG. 16 shows results of measuring tensions of hair over time, after applying a composition according to an embodiment (Example 2) to human hair.
FIG. 17 is a photograph of a human hair wig obtained by using a digital camera after applying a composition according to an embodiment (Example 1) thereto.
FIG. 18 is a photograph of a human hair wig obtained by using a digital camera after applying a composition according to an embodiment (Example 2) thereto.
FIG. 19 shows results of observing roughness of hair (cuticles) over time by using an electron microscope after applying a composition according to an embodiment (Example 1) to human hair.
FIG. 20 shows results observing roughness of hair (cuticles) over time by using an electron microscope after a composition according to an embodiment (Example 2) to human hair.

### Best Mode for Carrying out the Invention

Hereinafter, the present disclosure will be described in more detail with reference to the following examples. However, these examples are for illustrative purposes only, and the scope of the disclosure is not limited by these examples.

### Experimental Example 1. Synthesis of Peptide

Peptides shown in Table 1 below were synthesized using an automated peptide synthesizer (Milligen 9050, Millipore, USA), and pure peptides were isolated using a C18 reversed phase high performance liquid chromatography (HPLC, Waters Associates, USA). ACQUITY UPLC BEH300 C18 (2.1 mm × 100 mm, 1.7 µm, Waters Co, USA) was used as a column.

**Table 1**

| Name | Sequence (N-terminal -> C-terminal) |
|---|---|
| Peptide-1 | RCCG |
| Peptide-2 | EE |

### Experimental Example 2. Identification of Promotive Effect on Proliferation or Activation of Hair Follicle Dermal Papilla Cell

### 2-1. Identification of Promotive Effect on Proliferation of Hair Follicle Dermal Papilla Cell

In this experimental example, effects of a peptide according to an embodiment on proliferation of human hair follicle dermal papilla cells were examined by evaluating a change in viability of the human hair follicle dermal papilla cells (HFDPCs). Specifically, human hair follicle dermal papilla cells were seeded on a 96-well plate at a density of 4×10³ cells/well, followed by incubation in a mesenchymal stem cell complete medium for 24 hours. Subsequently, the culture medium was replaced with a serum free mesenchymal stem cell complete medium, followed by incubation for 24 hours. Thereafter, a peptide according to an embodiment was aliquoted thereinto at a concentration of 500 mM, 5 µM, or 50 µM, followed by incubation for 72 hours at 37 °C. Then, the culture was washed with PBS, and 10 µL of a 5 mg/ml MTT solution was aliquoted into each well. After incubating the culture in a CO₂ incubator for 4 hours, the culture medium was removed, and 100 µL of dimethyl sulfoxide (DMSO) was added thereto, followed by stirring for 10 minutes. Thereafter, absorbance was measured at a wavelength of 540 nm by using a spectrophotometer. Meanwhile, a untreated group was used as a control, and a group treated with 1 µM EGF was used as a positive control.

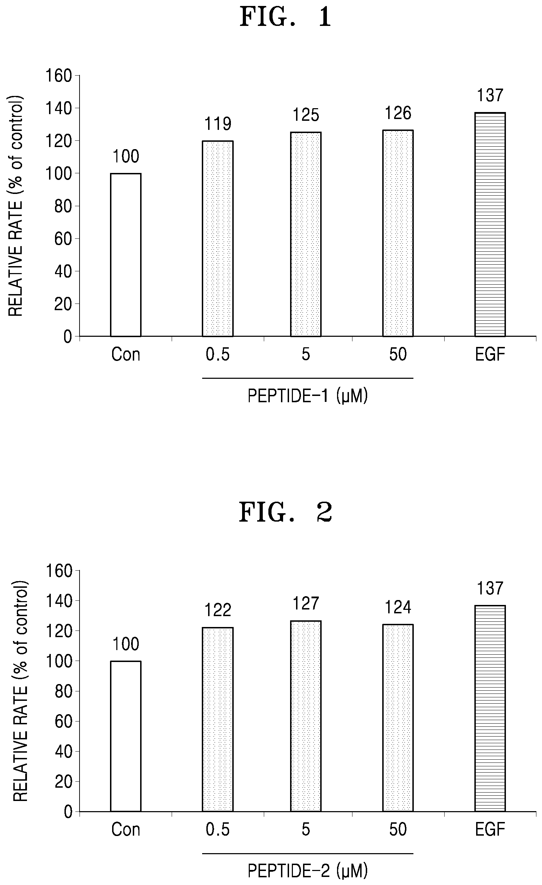

As a result, as shown in FIGS. 1 and 2, it was confirmed that proliferation of dermal papilla cells was promoted by Peptide-1 or Peptide-2.

### 2-2. Identification of Activating Effect on Factor Related to Proliferation of Hair Follicle Dermal Papilla Cell

In this experimental example, effects of a peptide according to an embodiment on activation of factors related to proliferation of human hair follicle dermal papilla cells were examined. Specifically, human hair follicle dermal papilla cells were seeded on a 6-well plate at a density of 4×10⁵ cells/well, followed by incubation in a mesenchymal stem cell complete medium for 24 hours. Thereafter, a peptide according to an embodiment was aliquoted thereinto at a concentration of 500 mM, 5 µM, or 50 µM, followed by incubation for 24 hours at 37 °C. Then, the culture was washed with PBS, and 100 µL of a lysis buffer to prepare a cell lysate. Subsequently, bicinchoninic acid (BCA) was quantified to prepare an equal amount of a protein sample, followed by electrophoresis using a 10 % SDS-PAGE gel. Thereafter, proteins separated by the SDS-PAGE were transferred to a PVDF membrane, and the reaction was blocked using a 5 % skim milk at room temperature for 30 minutes. Then, phospho-AKT and phospho-ERK (cell signaling, USA) antibodies were diluted in a 3 % BSA at 1:1000 and reacted with the blocked PVDF membrane at 4 °C for 16 hours. Subsequently, the reactants were washed with 0.1 % PBS-T (0.1 % Tween-20 in PBS) three times each for 15 minutes and reacted for 1 hour with a secondary antibody diluted with 5 % skim milk at 1:2000. Thereafter, the reactants were washed with 0.1 % PBS-T (0.1 % Tween-20 in PBS) three times each for 15 minutes and treated with an ECL solution (GE Healthcare, USA), and expression levels of the proteins were examined. Meanwhile, a untreated group was used as a control, and a group treated with 1 µM Minoxidil was used as a positive control.

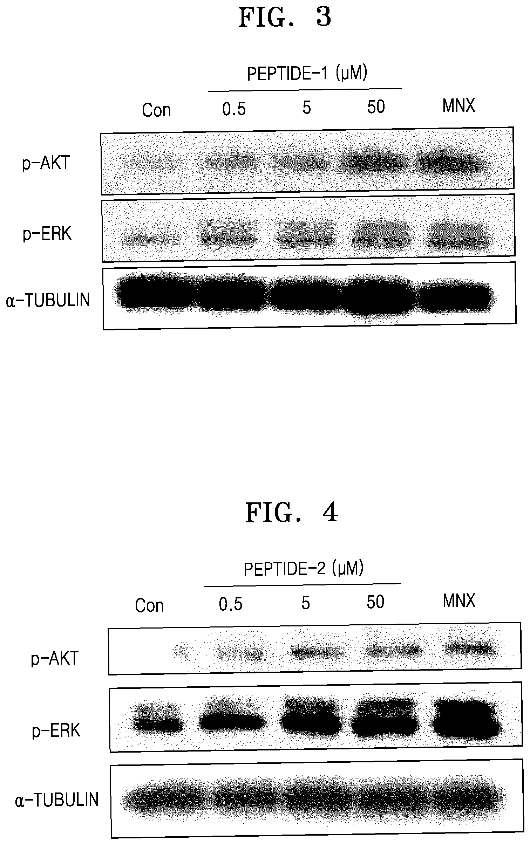

As a result, as shown in FIGS. 3 and 4, it was confirmed that phosphorylation of ERF and AKT, which are factors related to proliferation of hair follicle dermal papilla cells, was induced by Peptide-1 or Peptide-2.

### 2-3. Identification of Promotive Effect on Proliferation Mechanism of Hair Follicle Dermal Papilla Cell

In this experimental example, effects of a peptide according to an embodiment on proliferation mechanism of human hair follicle dermal papilla cells were examined. Specifically, human hair follicle dermal papilla cells were incubated in the same manner as in Experimental Example 2-2, and treated with a peptide according to an embodiment. Then, nuclear proteins were isolated from the culture by using a nuclear protein extraction kit (Thermo scientific, USA). Subsequently, expression levels of proteins were examined in the same manner as in Experimental Example 2-2 by using beta-catenin (cell signaling, USA) and HDAC1 (santa cruz, USA) antibodies. Meanwhile, a untreated group was used as a control, and a group treated with 10 ng/mL recombinant human Wnt-3a protein (rhWnt-3a, R&D systems (USA, MN)) was used as a positive control.

In addition, a change in expression levels of lymphoid enhancer-binding factor-1 (LEF-1), c-Myc, and cyclin D1, which are downstream target genes of activating mechanism of beta-catenin, was examined. To this end, human hair follicle dermal papilla cells were incubated in the same manner in Experimental Example 2-2 and treated with a peptide according to an embodiment. Thereafter, the culture was washed with PBS and treated with 300 µL of easy blue (intron, Korea) to extract RNAs. Then, the extract RNAs were quantified by Nano drop, and cDNA was synthesized by using the RNAs as a template and using a cDNA synthesis kit (enzynomics, Korea). Then, polymerase chain reaction (PCR) was carried out using primers of Table 2 below and a PCR premix (enzynomics, Korea), followed by electrophoresis using a 1.5 % agarose gel, and gene expression was examined in the RNA level by a Bio-Rad gel image system.

**Table 2**

| Primer | | Sequence (5'->3') | SEQ ID NO. |
|---|---|---|---|
| LEF-1 | Forward | CCAGCTATTGTAACACCTCA | 1 |
| | Reverse | TTCAGATGTAGGCAGCTGTC | 2 |
| c-Myc | Forward | ACCAGCAGCGACTCTGAGGAGGA AC | 3 |
| | Reverse | TGACCCTCTTGGCAGCAGGATAGT CC | 4 |
| cyclin-D1 | Forward | ACCTTCGTTGCCCTCTGTGCCACA GATG | 5 |
| | Reverse | AGGCCCGGAGGCAGTCCGGGT | 6 |
| GAPDH | Forward | GGAGCCAAAAGGGTCATCAT | 7 |
| | Reverse | GTGATGGCATGGACTGTGGT | 8 |

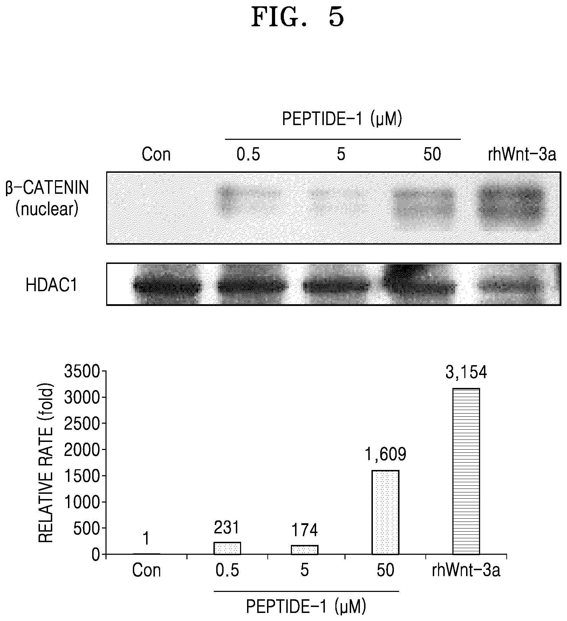

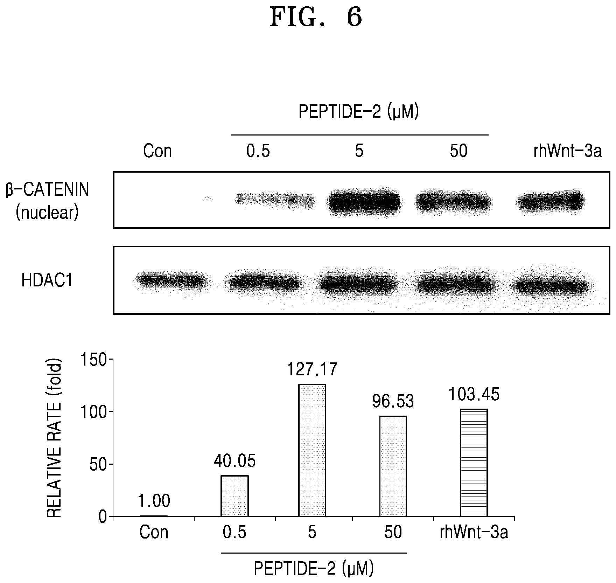

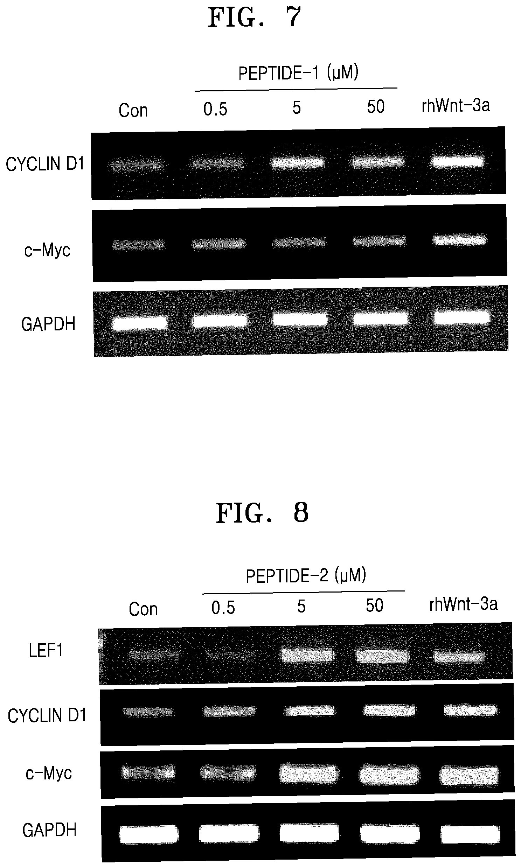

As a result, as shown in FIGS. 5 and 6, migration of beta-catenin from cytoplasm to a nucleus by Peptide-1 or Peptide-2 was confirmed. In addition, as shown in FIGS. 7 and 8, it was confirmed that beta-catenin was activated by Peptide-1 or Peptide-2, and accordingly, expression of LEF-1, c-Myc, and/or cyclin D1, which are downstream target genes, was increased.

### Experimental Example 3. Identification of Inhibitory Effect on Hair Loss-related Factor

In this experimental example, effects of a peptide according to an embodiment on expression of DKK-1 (Dickkopf-1), which is a hair loss inducer, were examined. Specifically, human hair follicle dermal papilla cells were incubated in the same manner in Experimental Example 2-2, and dihydrotestosterone (DHT), which is a substance inducing expression of DKK-1, was added thereto while aliquoting a peptide according to an embodiment thereinto at a concentration of 500 mM, 5 µM, or 50 µM. Subsequently, expression levels of proteins were examined in the same manner as in Experimental Example 2-2 by using DKK-1 (cell signaling, USA) antibodies. Meanwhile, a untreated group was used as a control, and a group treated with 5 µM Finasteride was used as a positive control.

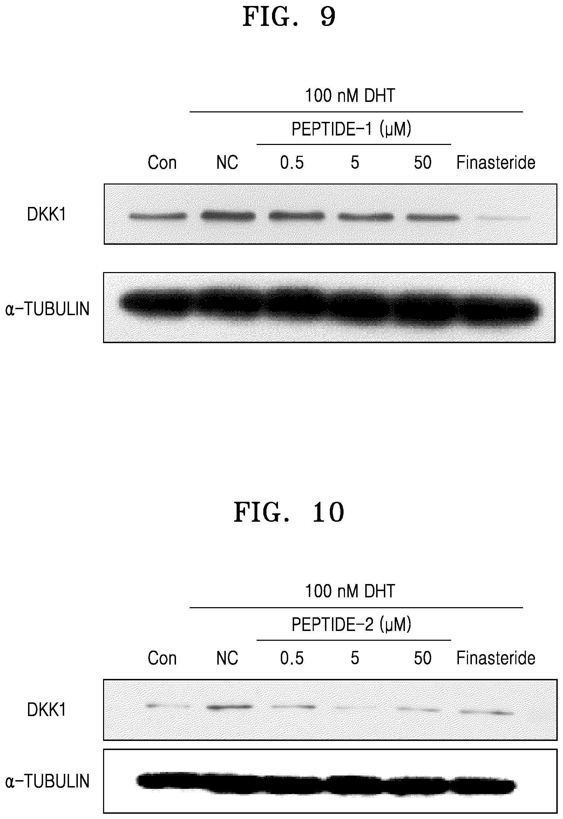

As a result, as shown in FIGS. 9 and 10, it was confirmed that expression of DKK-1, as the hair loss inducer, was inhibited by Peptide-1 or Peptide-2.

### Experimental Example 4. Identification of Promotive Effect on Activation of Outer Root Sheath Cell

In this experimental example, effects of a peptide according to an embodiment on expression of type I cuticular Ha3-II (Ha3-II), Keratin 5, Keratin 14, and Keratin 19, which are cytokine related to activation of human hair outer root sheath cells (HHORSCs), were examined. Specifically, human hair outer root sheath cells were seeded on a 6-well plate at a density of 4×10⁵ cell/well, followed by incubation for 24 hours in a mesenchymal stem cell complete media. Subsequently, the culture medium was replaced with a serum free mesenchymal stem cell complete medium, followed by incubation for 24 hours. Thereafter, a peptide according to an embodiment was aliquoted thereinto at a concentration of 500 mM, 5 µM, or 50 µM, followed by incubation for 24 hours at 37 °C. Then, gene expression was examined in the RNA level in the same manner as in Experimental Example 2-3 by using primers of Table 3 below. Meanwhile, a untreated group was used as a control, and a group treated with 50 nM EGF was used as a positive control.

**Table 3**

| Primer | | Sequence (5'->3') | SEQ ID NO. |
|---|---|---|---|
| Ha3-II | Forward | GATCATCGAGCTGAGACGCA | 9 |
| | Reverse | CTGGCCCCAGGGTATCTAGT | 10 |
| Keratin 5 | Forward | CCCTCAAGGATGCCAGGAAC | 11 |
| | Reverse | CACTGCTACCTCCGGCAAG | 12 |
| Keratin 14 | Forward | CCAAATCCGCACCAAGGTCA | 13 |
| | Reverse | GTATTGATTGCCAGGAGGGGG | 14 |
| Keratin 19 | Forward | CGCGGCGTATCCGTGTCCTC | 15 |
| | Reverse | AGCCTGTTCCGTCTCAAACTTGGT | 16 |
| GAPDH | Forward | GGAGCCAAAAGGGTCATCAT | 7 |
| | Reverse | GTGATGGCATGGACTGTGGT | 8 |

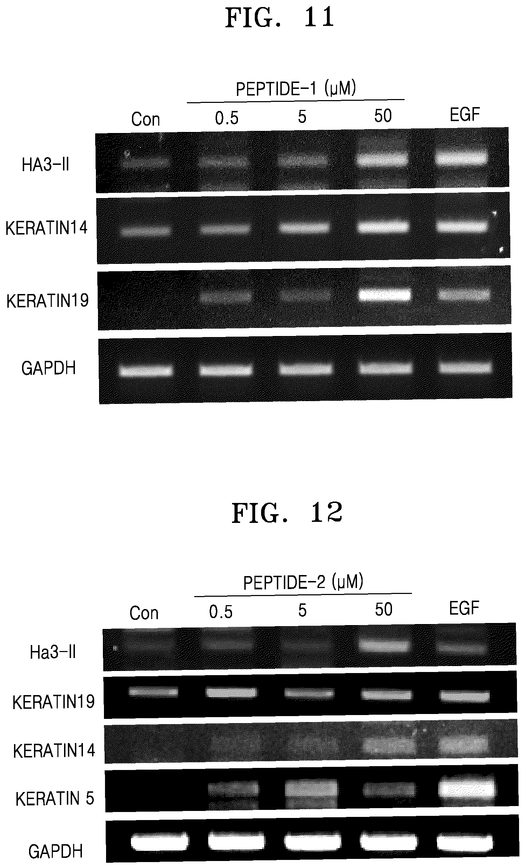

As a result, as shown in FIGS. 11 and 12, it was confirmed that expression of Ha3-II, Keratin 14, and Keratin 19, which are activators of outer root sheath cells, was increased by Peptide-1 or Peptide-2.

### Experimental Example 5. Identification of Promotive Effect on Activation of Germinal Matrix Cell

In this experimental example, effects of a peptide according to an embodiment on expression of Homeobox protein Hox-C13 (HOXC13), Msh homeobox 2 (MSX2), and Forkhead box protein N1 (FOXN1), which are transcription factors related to activation of human hair germinal matrix cells (HHGMCs), were examined. Specifically, human hair germinal matrix cells were seeded on a 6-well plate at a density of 4×10⁵ cell/well, followed by incubation for 24 hours in a mesenchymal stem cell complete medium. Subsequently, the culture medium was replaced with a serum free mesenchymal stem cell complete medium, followed by incubation for 24 hours. Thereafter, a peptide according to an embodiment was aliquoted thereinto at a concentration of 500 mM, 5 µM, or 50 µM, followed by incubation for 24 hours at 37 °C. Then, gene expression was examined in the RNA level in the same manner as in Experimental Example 2-3 by using primers of Table 4 below. Meanwhile, a untreated group was used as a control, and a group treated with 50 nM EGF was used as a positive control.

**Table 4**

| Primer | | Sequence (5'->3') | SEQ ID NO. |
|---|---|---|---|
| MSX2 | Forward | CGGTCAAGTCGGAAAATTCA | 17 |
| | Reverse | GAGGAGCTGGGATGTGGTAA | 18 |
| FOXN1 | Forward | CAGGACTGGGTGATGGTGTC | 19 |
| | Reverse | TCTGAGGGTGAGAGCCTGAA | 20 |
| GAPDH | Forward | GGAGCCAAAAGGGTCATCAT | 7 |
| | Reverse | GTGATGGCATGGACTGTGGT | 8 |

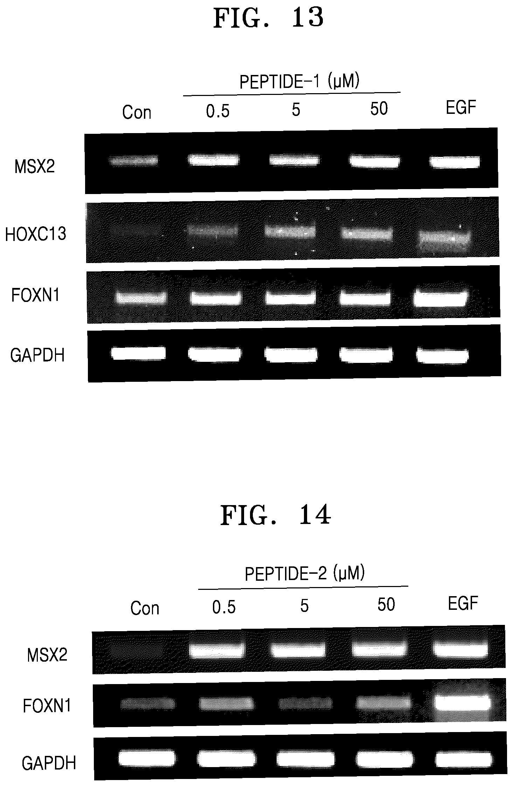

As a result, as shown in FIGS. 13 and 14, it was confirmed that expression of HOXC13, MSX2, and FOXN1, which are activators of germinal matrix cells, was increased by Peptide-1 or Peptide-2.

### Experimental Example 6. Preparation of Composition for Improving Condition of Damaged Hair

In this experimental example, a composition for improving condition of damaged hair including a peptide according to an embodiment (Peptide-1) as an active ingredient was prepared. Specifically, a composition of Example 1 was prepared in a mist formulation according to ingredients and contents shown in Table 5 below using a method known in the art. In addition, a composition of Example 2 was prepared in an emulsion formulation according to ingredients and contents shown in Table 6 below using a method known in the art.

**Table 5**

| | Ingredient | Content (wt%) |
|---|---|---|
| Example 1 | Peptide | 0.50 |
| | Arginine | 0.30 |
| | Cetrimonium chloride | 0.20 |
| | PEG-60 hydrogenated caster oil | 0.20 |
| | Citric acid | 0.05 |
| | Dipropylene glycol | 3.00 |
| | Ethanol | 9.50 |
| | Preservative | Appropriate amount |
| | Flavor | Appropriate amount |
| | Purified water | The balance |
| | Sum | 100 |

**Table 6**

| | Ingredient | Content (wt%) |
|---|---|---|
| Example 2 | Peptide | 0.20 |
| | Synthetic beeswax | 0.50 |
| | Polysolbate 20 | 0.10 |
| | Tocopheryl acetate | 0.10 |
| | Citric acid | 0.01 |
| | Dicaprylyl carbonate | 6.00 |
| | Dipropylene glycol | 5.00 |
| | Cetearyl alcohol | 4.00 |
| | Ethanol | 10.50 |
| | Preservative | Appropriate amount |
| | Flavor | Appropriate amount |
| | Purified water | The balance |
| | Sum | 100 |

### Experimental Example 7. Identification of Improving Effect on Tension of Damaged Hair

In this experimental example, effects of a peptide according to an embodiment on tension (elasticity) of damaged hair were examined. Specifically, 23 subjects were tested. After shampooing and drying hair once a day for 3 days, the composition of Example 1 or 2 was uniformly applied to the hair, and the hair was massaged, brushed, and maintained for 5 minutes. Then, hairs were collected from the occipital scalp of the subject and back load values (strength of a force when hair break, gmf) over time were measured by using a MTT175 (Miniature Tensile Tester, Diastron Ltd., UK).

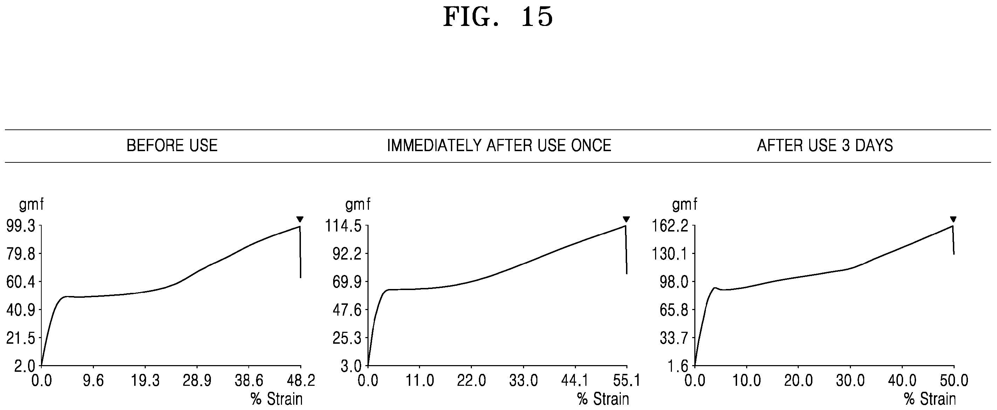

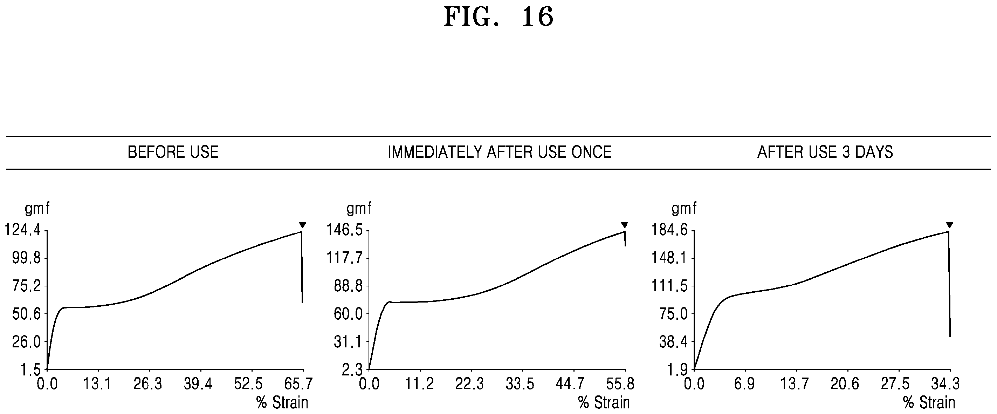

Results of Examples 1 and 2 are shown in Tables 7 and 8 below, and results of Examples 1 and 2 of one of the subjects are shown in FIGS. 15 and 16, respectively. These results indicate that the tension of hair increased as the break load value increased.

**Table 7**

| No. | Before use | Immediately after use once | After use for 3 days |
|---|---|---|---|
| 1 | 87.60 | 115.00 | 122.00 |
| 2 | 99.30 | 114.50 | 162.20 |
| 3 | 75.10 | 109.30 | 118.30 |
| 4 | 110.70 | 118.10 | 127.00 |
| 5 | 170.00 | 172.90 | 202.10 |
| 6 | 102.10 | 112.70 | 165.40 |
| 7 | 94.10 | 115.40 | 127.50 |
| 8 | 114.70 | 124.30 | 130.30 |
| 9 | 123.80 | 157.90 | 163.10 |
| 10 | 103.10 | 128.40 | 173.70 |
| 11 | 188.60 | 208.10 | 222.10 |
| 12 | 149.20 | 157.60 | 161.70 |
| 13 | 133.40 | 142.40 | 149.10 |
| 14 | 110.70 | 133.40 | 161.90 |
| 15 | 115.00 | 125.60 | 136.20 |
| 16 | 105.70 | 126.60 | 154.20 |
| 17 | 82.30 | 118.80 | 160.40 |
| 18 | 109.30 | 114.90 | 115.10 |
| 19 | 131.90 | 143.40 | 174.30 |
| 20 | 101.80 | 124.90 | 137.80 |
| 21 | 102.80 | 120.50 | 127.90 |
| 22 | 137.10 | 140.00 | 197.10 |
| 23 | 70.20 | 107.70 | 115.80 |
| Average | 113.85 | 131.84 | 152.40 |
| Standard deviation | 28.33 | 23.77 | 29.03 |

**Table 8**

| No. | Before use | Immediately after use once | After use for 3 days |
|---|---|---|---|
| 1 | 128.20 | 141.40 | 170.40 |
| 2 | 128.40 | 131.20 | 161.70 |
| 3 | 128.00 | 164.40 | 167.10 |
| 4 | 104.40 | 137.90 | 153.90 |
| 5 | 151.30 | 152.30 | 168.60 |
| 6 | 168.80 | 171.30 | 198.80 |
| 7 | 131.70 | 137.60 | 140.60 |
| 8 | 91.90 | 109.40 | 163.80 |
| 9 | 102.60 | 143.80 | 178.50 |
| 10 | 105.60 | 145.00 | 172.20 |
| 11 | 132.10 | 138.40 | 149.90 |
| 12 | 111.40 | 125.30 | 174.90 |
| 13 | 157.30 | 168.80 | 181.40 |
| 14 | 140.40 | 148.40 | 170.90 |
| 15 | 62.10 | 111.60 | 160.90 |
| 16 | 67.30 | 108.70 | 145.40 |
| 17 | 81.10 | 128.70 | 133.40 |
| 18 | 111.00 | 114.80 | 125.20 |
| 19 | 110.00 | 132.30 | 133.80 |
| 20 | 124.40 | 146.50 | 184.60 |
| 21 | 93.80 | 120.50 | 158.30 |
| 22 | 143.10 | 157.40 | 165.60 |
| 23 | 86.50 | 110.50 | 138.40 |
| Average | 115.71 | 136.79 | 160.80 |
| Standard deviation | 27.83 | 18.92 | 18.43 |

As a result, as shown in Tables 7 and 8, in the case of being treated with the composition of Example 1, it was confirmed that the average break load value was increased by 15.81 % immediately after use once and by 33.86 % after use for 3 days. In the case of being treated with the composition of Example 2, it was confirmed that the value was increased by 18.22 % immediately after use once and by 38.96 % after used for 3 days. In comparison with the results before use, statistically significant differences were observed by Examples 1 and 2 in both cases of immediately after use once and after use for 3 days (p<0.001).

Based on the results, it was confirmed the peptide according to an embodiment had effects on increasing tension (elasticity) of damaged hair.

### Experimental Example 8. Identification of Improving Effect on Gloss of Hair

In this experimental example, effects of a peptide according to an embodiment on gloss (angel ring) of damaged hair were examined. Specifically, 20 human hair wigs were tested. After shampooing and drying the human hair wig, the composition of Example 1 or 2 was uniformly applied to the human hair wig, and the wig was massaged, brushed, and maintained for 5 minutes. Then, gloss unit values were measured using a glossmeter (Multi Gloss 268 PLUS, Konica Minolta, Japan) at 60°.

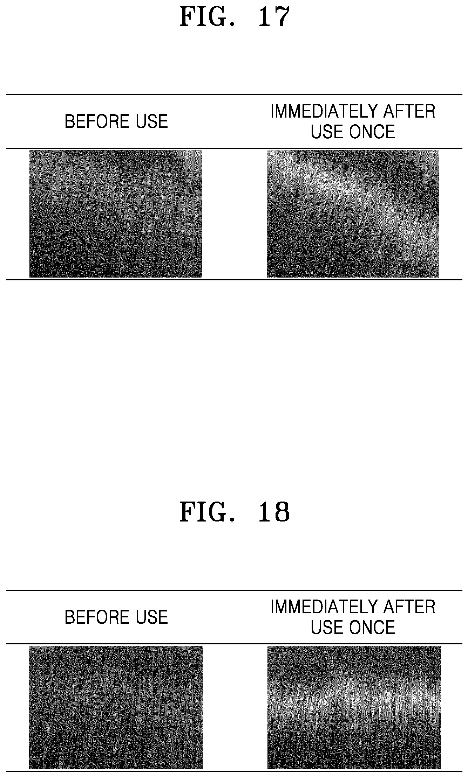

The results of Examples 1 and 2 are shown in Tables 9 and 10, respectively, and indicate that gloss of hair increased as the gloss unit value increased. In addition, photographs of one of the human hair wigs were obtained by using a digital camera after the compositions of Examples 1 and 2 were applied thereto, and the results are shown in FIGS. 17 and 18.

**Table 9**

| No. | Before use | Immediately after use once |
|---|---|---|
| 1 | 0.21 | 0.33 |
| 2 | 0.27 | 0.31 |
| 3 | 0.24 | 0.33 |
| 4 | 0.22 | 0.46 |
| 5 | 0.22 | 0.39 |
| 6 | 0.23 | 0.37 |
| 7 | 0.25 | 0.44 |
| 8 | 0.31 | 0.50 |
| 9 | 0.20 | 0.39 |
| 10 | 0.21 | 0.44 |
| 11 | 0.24 | 0.50 |
| 12 | 0.19 | 0.34 |
| 13 | 0.26 | 0.44 |
| 14 | 0.21 | 0.46 |
| 15 | 0.19 | 0.43 |
| 16 | 0.25 | 0.48 |
| 17 | 0.22 | 0.42 |
| 18 | 0.24 | 0.45 |
| 19 | 0.25 | 0.31 |
| 20 | 0.20 | 0.45 |
| Average | 0.23 | 0.41 |
| Standard deviation | 0.03 | 0.06 |

**Table 10**

| No. | Before use | Immediately after use once |
|---|---|---|
| 1 | 0.24 | 0.42 |
| 2 | 0.26 | 0.38 |
| 3 | 0.24 | 0.49 |
| 4 | 0.21 | 0.36 |
| 5 | 0.24 | 0.49 |
| 6 | 0.23 | 0.42 |
| 7 | 0.25 | 0.50 |
| 8 | 0.27 | 0.44 |
| 9 | 0.25 | 0.51 |
| 10 | 0.25 | 0.36 |
| 11 | 0.23 | 0.51 |
| 12 | 0.20 | 0.41 |
| 13 | 0.23 | 0.36 |
| 14 | 0.28 | 0.54 |
| 15 | 0.24 | 0.36 |
| 16 | 0.27 | 0.38 |
| 17 | 0.23 | 0.41 |
| 18 | 0.21 | 0.50 |
| 19 | 0.23 | 0.43 |
| 20 | 0.24 | 0.46 |
| Average | 0.24 | 0.44 |
| Standard deviation | 0.02 | 0.06 |

As a result, as shown in Tables 10 and 11, in the case of being treated with the composition of Example 1, it was confirmed that the average gloss unit value was increased by 78.61 % immediately after used once. In the case of being treated with the composition of Example 2, it was confirmed that the average gloss unit value was increased by 82.15 % immediately after used once. In comparison with the results before use, statistically significant differences were observed by Examples 1 and 2 in the case of immediately after use once (p<0.001).

Based on the results, it was confirmed the peptide according to an embodiment had effects on increasing gloss (angel ring) of damaged hair.

### Experimental Example 9. Identification of Alleviating Effect on Roughness of Damaged Hair

In this experimental example, effects of a peptide according to an embodiment on roughness (cuticles) of damaged hair were examined. Specifically, 23 subjects were tested using the composition of Example 1 or 2 in the same manner as in Experimental Example 7. Images of hair of the occipital scalp of the subject were obtained by using a scanning electron microscope (S-4700, Hitachi, Japan) and magnified 700 times, and then a variable Ra (µm) indicating roughness of surface of hair over time was measured using an image analysis program (Image J, National Institutes of Health, USA).

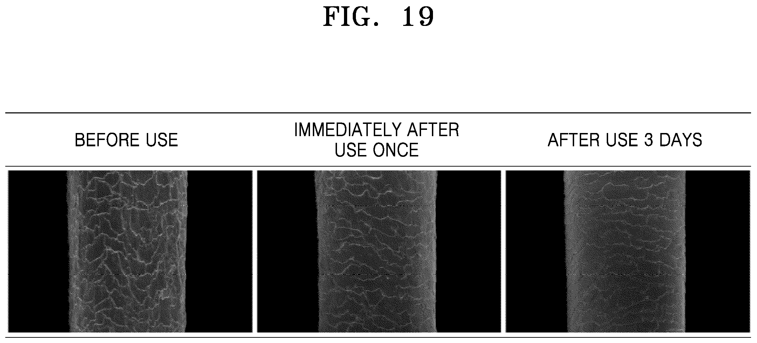

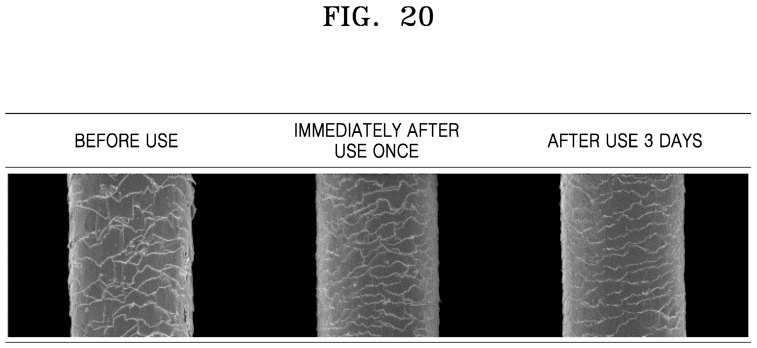

Results of Examples 1 and 2 are shown in Tables 11 and 12 below, respectively and indicate that cuticles were improved and roughness of hair were reduced as the Ra value increased. In addition, results of imaging the hair of one of the subjects treated according to Examples 1 and 2 by using an electron microscope are shown in FIGS. 19 and 20, respectively.

**Table 11**

| No. | Before use | Immediately after use once | After use for 3 days |
|---|---|---|---|
| 1 | 18.40 | 17.86 | 14.86 |
| 2 | 20.95 | 14.96 | 15.82 |
| 3 | 20.64 | 14.01 | 10.65 |
| 4 | 21.48 | 14.82 | 10.39 |
| 5 | 25.11 | 16.22 | 16.46 |
| 6 | 21.47 | 20.34 | 13.72 |
| 7 | 20.86 | 12.98 | 10.25 |
| 8 | 22.91 | 23.45 | 13.08 |
| 9 | 23.71 | 20.52 | 10.81 |
| 10 | 19.02 | 15.09 | 11.85 |
| 11 | 21.48 | 17.30 | 13.71 |
| 12 | 19.59 | 13.20 | 12.07 |
| 13 | 19.76 | 15.54 | 14.89 |
| 14 | 20.22 | 16.76 | 12.05 |
| 15 | 22.06 | 19.40 | 13.45 |
| 16 | 21.12 | 15.94 | 17.70 |
| 17 | 18.89 | 17.93 | 16.37 |
| 18 | 19.70 | 14.27 | 13.14 |
| 19 | 25.64 | 16.63 | 17.78 |
| 20 | 21.84 | 13.01 | 11.99 |
| 21 | 24.54 | 23.95 | 11.88 |
| 22 | 18.48 | 17.64 | 12.78 |
| 23 | 17.13 | 15.99 | 16.44 |
| Average | 21.09 | 16.86 | 13.57 |
| Standard deviation | 2.20 | 3.03 | 2.33 |

**Table 12**

| No. | Before use | Immediately after use once | After use for 3 days |
|---|---|---|---|
| 1 | 22.41 | 19.08 | 12.64 |
| 2 | 24.32 | 20.85 | 17.53 |
| 3 | 27.68 | 21.12 | 12.05 |
| 4 | 23.57 | 17.36 | 15.69 |
| 5 | 22.99 | 17.92 | 13.46 |
| 6 | 24.88 | 19.32 | 10.27 |
| 7 | 21.46 | 20.34 | 12.21 |
| 8 | 25.94 | 24.53 | 19.41 |
| 9 | 20.37 | 16.12 | 14.16 |
| 10 | 24.03 | 17.94 | 14.84 |
| 11 | 23.53 | 16.01 | 14.36 |
| 12 | 21.35 | 16.77 | 14.79 |
| 13 | 22.97 | 14.71 | 14.76 |
| 14 | 25.93 | 15.64 | 15.24 |
| 15 | 27.73 | 16.11 | 14.90 |
| 16 | 20.45 | 13.24 | 12.06 |
| 17 | 21.74 | 13.11 | 10.11 |
| 18 | 23.23 | 20.60 | 17.07 |
| 19 | 24.85 | 19.50 | 15.49 |
| 20 | 23.62 | 17.51 | 15.73 |
| 21 | 20.78 | 18.41 | 17.89 |
| 22 | 22.32 | 16.28 | 13.33 |
| 23 | 22.72 | 19.28 | 16.46 |
| Average | 23.43 | 17.90 | 14.54 |
| Standard deviation | 2.07 | 2.69 | 2.34 |

As a result, as shown in Tables 11 and 12, in the case of being treated with the composition of Example 1, it was confirmed that the average Ra value was decreased by 20.04 % immediately after use once and by 35.64 % after use for 3 days. In the case of being treated with the composition of Example 2, it was confirmed that the Ra value was decreased by 23.59 % immediately after use once and by 37.94 % after used for 3 days. In comparison with the results before use, statistically significant differences were observed by Examples 1 and 2 in both cases of immediately after use once and after use for 3 days (p<0.001).

Based on the results, it was confirmed that the peptide according to an embodiment had effects on alleviating roughness (cuticles) of damaged hair.

### Experimental Example 10. Identification of Reducing Effect on Friction of Damaged Hair

In this experimental example, effects of a peptide according to an embodiment on friction of damaged hair were examined. Specifically, the composition of Example 1 or 2 was applied to human hair pieces (bleached hair) in the same manner as in Experimental Example 8, except that 20 human hair pieces (bleached hair) were used. Then, the human hair pieces (bleached hair) were evaluated by measuring a Mean Horizontal Force Out value (gmf/mm) indicating an average horizontal friction of hair of a hair root area by using a MTT175 (Miniature Tensile Tester, Dia-stron Ltd., UK).

Results of Examples 1 and 2 are shown in Tables 13 and 4, respectively. The results indicate that friction of hair was reduced as the Mean Horizontal Force Out value decreased.

**Table 13**

| No. | Before use | Immediately after use once |
|---|---|---|
| 1 | 96.13 | 58.63 |
| 2 | 158.20 | 152.30 |
| 3 | 105.60 | 97.28 |
| 4 | 101.60 | 68.30 |
| 5 | 104.00 | 100.30 |
| 6 | 54.24 | 37.37 |
| 7 | 118.90 | 69.48 |
| 8 | 95.70 | 53.57 |
| 9 | 113.90 | 70.50 |
| 10 | 113.10 | 97.47 |
| 11 | 65.53 | 43.99 |
| 12 | 100.40 | 49.79 |
| 13 | 100.10 | 83.20 |
| 14 | 118.10 | 99.35 |
| 15 | 102.80 | 62.16 |
| 16 | 87.78 | 68.73 |
| 17 | 101.70 | 70.38 |
| 18 | 94.08 | 59.51 |
| 19 | 109.50 | 74.54 |
| 20 | 118.70 | 88.21 |
| Average | 103.00 | 75.25 |
| Standard deviation | 20.85 | 25.91 |

**Table 14**

| No. | Before use | Immediately after use once |
|---|---|---|
| 1 | 119.00 | 64.62 |
| 2 | 117.30 | 61.97 |
| 3 | 133.20 | 78.48 |
| 4 | 145.30 | 115.47 |
| 5 | 75.09 | 58.23 |
| 6 | 102.50 | 83.89 |
| 7 | 82.54 | 65.59 |
| 8 | 136.80 | 89.77 |
| 9 | 141.50 | 105.92 |
| 10 | 142.80 | 119.79 |
| 11 | 128.50 | 71.38 |
| 12 | 137.60 | 108.15 |
| 13 | 151.90 | 124.15 |
| 14 | 152.00 | 90.77 |
| 15 | 133.50 | 67.72 |
| 16 | 132.80 | 99.06 |
| 17 | 127.20 | 71.85 |
| 18 | 120.90 | 103.24 |
| 19 | 124.60 | 65.39 |
| 20 | 128.20 | 100.90 |
| Average | 126.66 | 87.32 |
| Standard deviation | 20.31 | 21.15 |

As a result, as shown in Tables 13 and 14, in the case of being treated with the composition of Example 1, it was confirmed that the average Mean Horizontal Force Out value was reduced by 26.94 % immediately after use once. In the case of being treated with the composition of Example 2, it was confirmed that the average Mean Horizontal Force Out value was reduced by 31.06 % immediately after use once. In comparison with the results before use, statistically significant differences were observed by Examples 1 and 2 in both cases of immediately after use once (p<0.001).

Based on the results, it was confirmed the peptide according to an embodiment had effects on reducing friction of damaged hair.

### Experimental Example 11. Evaluation of Abnormal Skin Reaction

Occurrence of abnormal reaction by the composition was observed on the 23 subjects of Experimental Example 7 described above. As a result, it was confirmed that no allergic contact dermatitis or no irritant contact dermatitis were observed. In addition, the subjects were asked to report erythema (redness), edema (swelling), scale (dead skin cells), itching, stinging (pain), burning, stiffness, and tingling. As a result, abnormal skin reaction was not reported.

Based on the results, it was confirmed that the composition including the peptide according to an embodiment was suitable for application to skin.

### Formulation Example 1. Preparation of Peptide Nanosomes

50 mg of the peptide of Example 1 was dissolved in 500 ml of distilled water by sufficiently stirring. The mixture solution was mixed with 5 g of lecithin, 0.3 ml of sodium oleate, 50 ml of ethanol, and a slight amount of an oil phase, and then distilled water was added thereto to adjust a total amount to 1 L. Then, the mixture was emulsified by using a microfluidizer at high pressure to prepare peptide nanosomes having a size of 100 nm.

### Formulation Example 2. Lotion

A lotion including the peptide according to an embodiment and the following composition was prepared according to a method known in the art.

**Table 15**

| Ingredient | Content (wt%) |
|---|---|
| Peptide | 1.0 |
| 1,3-butylene glycol | 6.0 |
| Glycerin | 4.0 |
| PEG 1500 | 1.0 |
| Sodium hyaluronate | 1.0 |
| Polysolbate 20 | 0.5 |
| Ethanol | 8.0 |
| Benzophenone-9 | 0.05 |
| Preservative, pigment | Appropriate amount |
| Flavor | Appropriate amount |
| Purified water | The balance |
| Sum | 100 |

### Formulation Example 3. Nourishing cream

A nourishing cream including the peptide according to an embodiment and the following composition was prepared according to a method known in the art.

**Table 16**

| Ingredient | Content (wt%) |
|---|---|
| Peptide | 2.0 |
| Glycerin | 10.0 |
| 1,3-butylene glycol | 5.0 |
| PEG 1500 | 2.0 |
| Allantoin | 0.1 |
| DL-panthenol | 0.3 |
| EDTA-2Na | 0.02 |
| Hydroxyethyl cellulose | 0.1 |
| Sodium hyaluronate | 8.0 |
| Carboxy vinyl polymer | 0.2 |
| Triethanolamine | 0.18 |
| Octyldodeceth-16 | 0.4 |
| Ethanol | 6 |
| Preservative, pigment | Appropriate amount |
| Flavor | Appropriate amount |
| Purified water | The balance |
| Sum | 100 |

The above description of the present disclosure is provided for the purpose of illustration, and it would be understood by those skilled in the art that various changes and modifications may be made without changing technical conception and essential features of the present disclosure. Thus, it is clear that the above-described embodiments of the present disclosure are illustrative in all aspects and do not limit the present disclosure.

## Claims

1. A peptide consisting of an amino acid sequence represented by Arg (R)-Cys (C)-Cys (C)-Gly (G) or Glu (E)-Glu (E).

2. The peptide of claim 1, wherein a N-terminal of the peptide is bound to one protective group selected from an acetyl group, a fluoreonylmethoxycarbonyl group, a formyl group, a palmitoyl group, a myristyl group, a stearyl group, a butoxycarbonyl group, an allyloxycarbonyl group, and a polyethylene glycol (PEG).

3. The peptide of claim 1, wherein a C-terminal of the peptide is bound to one protective group selected from an amino group (-NH₂), a tertiary alkyl group, and an azide group (-NHNH₂).

4. The peptide of claim 1, wherein the peptide exhibits at least one characteristic selected from the following characteristics:
(a) promoting proliferation or activation of hair follicle dermal papilla cells;
(b) promoting activation of outer root sheath cells;
(c) promoting activation of germinal matrix cells; and
(d) inhibiting expression of Dickkopf-related protein 1 (DKK-1).

5. A cosmetic composition for improving condition of damaged hair comprising a peptide including an amino acid sequence represented by Arg (R)-Cys (C)-Cys (C)-Gly (G) or Glu (E)-Glu (E) as an active ingredient.

6. A cosmetic composition for alleviating hair loss or promoting hair growth comprising a peptide including an amino acid sequence represented by Arg (R)-Cys (C)-Cys (C)-Gly (G) or Glu (E)-Glu (E) as an active ingredient.

7. A pharmaceutical composition for preventing or treating hair loss comprising a peptide including an amino acid sequence represented by Arg (R)-Cys (C)-Cys (C)-Gly (G) or Glu (E)-Glu (E) as an active ingredient.
